# EUROPÄISCHE PATENTSCHRIFT

(11) **EP 3 086 747 B1**
(45) Veröffentlichungstag und Bekanntmachung des Hinweises auf die Patenterteilung: **07.07.2021**
(21) Anmeldenummer: 14830937.0
(22) Anmeldetag: 19.11.2014
(51) Int. Cl.: A61F 13/15

(54) **VORRICHTUNG UND VERFAHREN ZUR HERSTELLUNG EINES KOMPOSITS**
DEVICE AND METHOD FOR PRODUCING A COMPOSITE
DISPOSITIF ET PROCÉDÉ DE PRODUCTION D'UN COMPOSITE

(30) Priorität: 20.11.2013 DE 102013019314; 20.11.2013 DE 102013019316
(43) Veröffentlichungstag der Anmeldung: 02.11.2016
(73) Patentinhaber: IMECO GMBH & CO. KG, 63768 Hösbach (DE)
(72) Erfinder: HOLTMANN, Michael, 63768 Hösbach (DE)
(74) Vertreter: Nitz, Astrid
(86) Internationale Anmeldenummer: PCT/DE2014/000592
(87) Internationale Veröffentlichungsnummer: WO 2015/074639

(56) Entgegenhaltungen:
- EP-A1- 2 412 346
- EP-A1- 2 586 410
- WO-A1-2007/020562
- None

## Beschreibung

Die Erfindung betrifft eine Vorrichtung zur Herstellung eines Komposits umfassend Partikel und/oder Pulver, insbesondere Absorberpartikel und/oder Partikel zur Wirkstoffabgabe nach dem Oberbegriff von Anspruch 1 und einem Verfahren zur Herstellung eines Komposits umfassend Partikel und/oder Pulver, insbesondere Absorberpartikel nach dem Oberbegriff von Anspruch 9.

Bekannt sind Pulver, die auf verschiedene Arten in Materialbahnen einzuschließen und als Komposit zu fixieren sind. Das Pulver kann verschiedene Aufgaben erfüllen, beispielsweise als Superabsorber Flüssigkeiten aufnehmen.

Bekannt ist es, das Pulver mit einem pulverförmigen Heißkleber zu verbinden und dieses auf die Materialbahn zu streuen, diese Materialbahn dann mit einer zweiten Materialbahn abzudecken, und diesen Verbund dann mittels thermischer Beaufschlagung mit einem Heißklebesystem zu befestigen. Nachteilig ist es, dass dabei aufgrund der undefinierten Vermischung der beiden eingesetzten Pulver Randbereiche soweit offen bleiben können, dass Pulver an den Außenkanten austreten kann.

Bekannt ist es weiterhin, mittels undefinierten Auftrags locker aufgebrachtes Pulver auf bahnförmigem Material partiell oder flächig zu verschweißen. Nachteilig ist dabei, dass immer Pulver in die Schweißnaht gerät und dann beim Aufquellen für Undichtigkeiten sorgt.

Aufgabe der vorliegenden Erfindung ist es, eine Vorrichtung bereitzustellen, die eine sichere Herstellung von Komposit in einen sicheren, wartungsarmen Betrieb mit einem hohen Wirkungsgrad ermöglicht.

Die Aufgabe wird gelöst durch eine Vorrichtung zur Herstellung eines Komposits umfassend Partikel und/oder Pulver, insbesondere Absorberpartikel und/oder Partikel zur Wirkstoffabgabe, sowie mindestens zwei Materiallagen, umfassend eine zumindest teilweise rotierbare Walze mit Unterdruckbereichen, ein erstes und ein zweites Versorgungsmittel, ein Mittel zur Zuführung, ein Mittel zum Verbinden, wobei auf der Walze eine erste Materiallage mittels des ersten Versorgungsmittels aufzulegen ist und die Walze an vorbestimmten Bereichen mit Unterdruck beaufschlagt ist zum Ansaugen der Materiallagen und/oder von Partikeln und/oder Pulver, die mittels des in einem vorbestimmten Anlagebereich zur Walze angeordneten Mittels zur Zuführung derart an den vorbestimmten Bereichen anzubieten sind, dass durch den Unterdruck die vorbestimmten Bereiche auf der Materiallage mit Partikel und/oder Pulver zumindest teilweise bedeckt sind und mittels eines zweiten, an der Walze angeordneten Versorgungsmittels eine zweite Materiallage auf die erste Materiallage mit Partikeln und/oder Pulver aufzudecken ist, wobei diese in vorbestimmten Bereichen, die insbesondere zwischen den Unterdruckbereichen angeordnet sind, mit der ersten Materiallage insbesondere durch Einwirken des Mittels zum Verbinden, das an der Walze angeordnet ist, verbunden ist, so dass das Komposit zumindest eine im Wesentlichen geschlossene Kammer umfassend Partikel und/oder Pulver aufweist, wobei die zumindest teilweise rotierende Walze eine insbesondere feststehende Welle aufweist. wobei die Partikel und/oder Pulver und/oder die Materiallage, insbesondere abgetrennt von und/oder ausgestanzt aus flächigem, insbesondere bahnförmigem Material, mittels eines der Welle zugeordneten Mittels zur Druck- und/oder Unterdruckerzeugung beaufschlagt ist, nämlich einem Mittel unter Ausnutzung des Bernoullieffekts, nämlich einer oder mehrerer Venturidüsen, direkt oder indirekt durch Transportmittel, insbesondere Transportdüsen, zur Druckweiterleitung aus dem Bereich der Welle, insbesondere an dem Unterdruckbereich, durch ein rotierendes Mantelmittel hindurch manipuliert sind, so dass insbesondere in einem vorbestimmten lokalen Bereich des Mantelmittels die Materiallage und/oder Partikel und/oder Pulver mittels Unterdruck festgehalten und/oder mittels Überdruck abgestoßen werden -

Die Vorrichtung ermöglicht durch die Ansaugung der Partikel und/oder des Pulvers durch Unterdruck durch die Materiallage hindurch eine genaue und abgegrenzte Anordnung der Partikel und/oder des Pulvers, ohne dass diese durch eine zufällige Verteilung in Bereiche der Materiallagen geraten, die verbunden werden. Die Dosierung der Ansaugung und die Verteilung der Partikel und /oder des Pulvers ist individuell einstellbar und sicher zu steuern.

Vorteilhaft ist es, wenn die zumindest teilweise rotierende Walze eine insbesondere feststehende Welle aufweist, wobei die Partikel und/oder Pulver und/oder die Materiallage, insbesondere abgetrennt von und/oder ausgestanzt aus flächigem, insbesondere bahnförmigem Material, mittels eines der Welle zugeordneten Mittels zur Druck- und/oder Unterdruckerzeugung beaufschlagt ist, insbesondere einer Vakuumpumpe und/oder einem Mittel unter Ausnutzung des Bernoullieffekts, insbesondere einer oder mehrerer Venturidüsen, direkt oder indirekt durch Transportmittel, insbesondere Transportdüsen, zur Druckweiterleitung aus dem Bereich der Welle, insbesondere an dem Unterdruckbereich, durch ein rotierendes Mantelmittel hindurch manipuliert sind, so dass insbesondere in einem vorbestimmten lokalen Bereich des Mantelmittels die Materiallage und/oder Partikel und/oder Pulver mittels Unterdruck festgehalten und/oder mittels Überdruck abgestoßen werden.

Vorteilhaft ist es, wenn die Walze am Mantelmittel mit einer Struktur versehen ist, die die Begrenzungen der Kammern mit den Partikeln und/der Pulver ausformen.

Vorteilhaft ist es, wenn das erste und/oder zweite Versorgungsmittel Rollen zum Abrollen der Materiallagen umfassen.

Vorteilhaft ist es, wenn das Mittel zur Zuführung für die Partikel und/oder das Pulver hinsichtlich der Rotationsrichtung nach dem Auflegen der ersten Materiallage und vor dem Auflegen der zweiten Materiallage angeordnet ist und/oder das Mittel zum Verbinden hinsichtlich der Rotationsrichtung der Walze nach dem Aufsetzpunkt der zweiten Materiallage angeordnet ist.

Vorteilhaft ist es, wenn das Mittel zur Zuführung ein Lieferband umfasst, das die Partikel und/oder das Pulver in einer Spur zu der Walze transportiert, wobei durch eine Mittel zur Zuführung insbesondere eine vorbestimmte bahnartige Besetzung des Lieferbandes vorzunehmen ist.

Vorteilhaft ist es, wenn das Mittel zur Zuführung eine insbesondere kastenartige Befülleinrichtung umfasst, die unterhalb der Walze angerordnet ist, wobei der Unterdruck Partikel und/oder Pulver aus der Befülleinrichtung herausheben kann und/oder das Mittel zur Zuführung eine Förderschnecke umfasst, die insbesondere an einer Oberfläche Löcher im Bereich von Transportdüsen aufweist, wobei diese Löcher auch als durchgehender Schlitz ausgeführt sein können.

Vorteilhaft ist es, wenn das Mittel zum Verbinden eine Klebeeinrichtung und/oder eine Einrichtung zum Ultraschallschweißen und/oder eine Temperaturschweißvorrichtung umfasst.

Vorteilhaft ist es, wenn die Materiallage umfasst Polymer und/oder Vlies und/oder Folie und/oder Gewebe und/oder Gewirke und/oder ähnliche Flächengebilde, die insbesondere zur Weiterleitung von Druckänderungen geeignet sind und/oder flüssigkeitsdurchlässig sind.

Die Aufgabe wird ebenfalls gelöst durch ein Verfahren zur Herstellung eines Komposits umfassend Partikel und/oder Pulver, insbesondere Absorberpartikel, insbesondere nach einem der Ansprüche 1 bis 8, sowie mindestens zwei Materiallagen, wobei eine Materiallage auf einer zumindest teilweise rotierenden Walze mit vorbestimmten Unterdruckbereichen bereitgestellt wird und diesen Unterdruckbereichen Partikel und/oder Pulver zum Ansaugen durch den Unterdruck mittels eines Mittel zur Zuführung angeboten werden, so dass durch den Unterdruck die vorbestimmten Bereiche auf der Materiallage mit Partikeln und/oder Pulver im Wesentlichen bedeckt werden und anschließend mittels eines zweiten Versorgungsmittels eine zweite Materiallage auf die erste Materiallage mit den Partikeln und/oder Pulver aufgelegt wird und in vorbestimmten Bereichen, die insbesondere zwischen den Unterdruckbereichen angeordnet sind, mit der ersten Materiallage insbesondere durch Einwirken eines Mittels zum Verbinden, das an der Walze angeordnet ist, verbunden werden, so dass zumindest eine oder mehrere im Wesentlichen geschlossene Kammer umfassend Partikel und/oder Pulver hergestellt wird,
wobei die zumindest teilweise rotierende Walze eine insbesondere feststehende Welle aufweist, wobei die Partikel und/oder Pulver und/oder die Materiallage, insbesondere abgetrennt von und/oder ausgestanzt aus flächigem, insbesondere bahnförmigem Material, mittels eines der Welle zugeordneten Mittels zur Druck- und/oder Unterdruckerzeugung beaufschlagt ist, nämlich einem Mittel unter Ausnutzung des Bernoullieffekts, nämlich einer oder mehrerer Venturidüsen, direkt oder indirekt durch Transportmittel, insbesondere Transportdüsen, zur Druckweiterleitung aus dem Bereich der Welle, insbesondere an dem Unterdruckbereich, durch ein rotierendes Mantelmittel hindurch manipuliert sind, so dass insbesondere in einem vorbestimmten lokalen Bereiches Mantelmittels die Materiallage und/oder Partikel und/oder Pulver mittels Unterdruck festgehalten und/oder mittels Überdruck abgestoßen werden.

Weitere Merkmale und Vorteile der Erfindung ergeben sich aus den Ansprüchen und der nachstehenden Beschreibung, in der Ausführungsbeispiele des Gegenstands der Erfindung in Verbindung mit den Zeichnungen näher erläutert sind.

Es zeigen:
- Fig. 1: eine erfindungsgemäße Vorrichtung in perspektivischer Ansicht,
- Fig. 2: eine Walze in Explosionsdarstellung,
- Fig. 3: eine Partikel- und/oder Pulveraufhäufung,
- Fig. 4: eine erfindungsgemäße Vorrichtung,
- Fig. 5: eine erfindungsgemäße Vorrichtung und
- Fig. 6: ein Komposit.

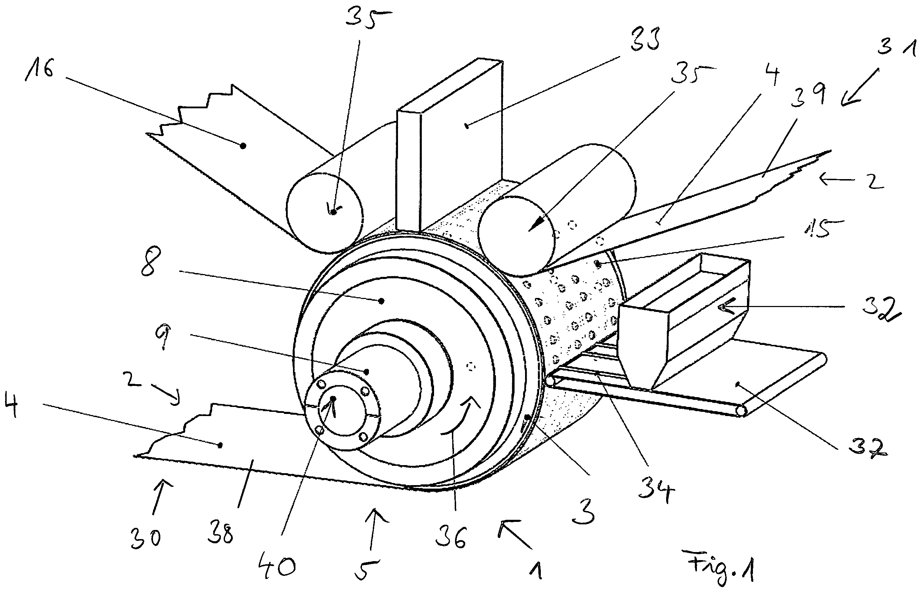

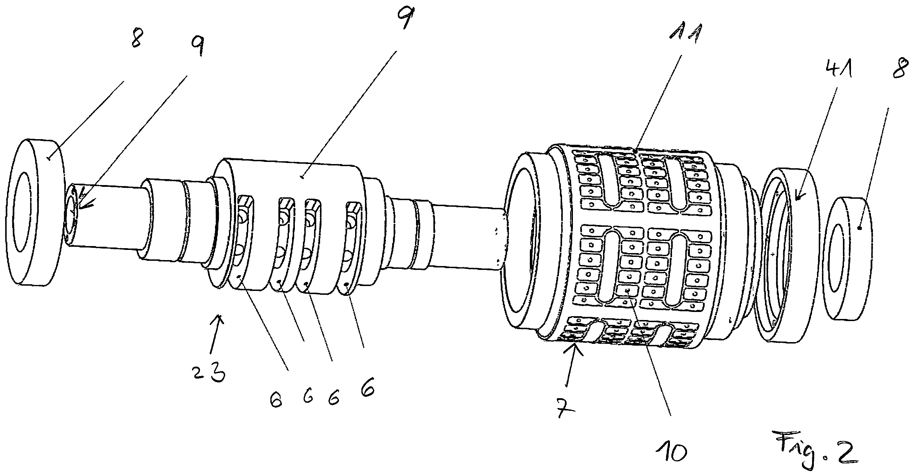

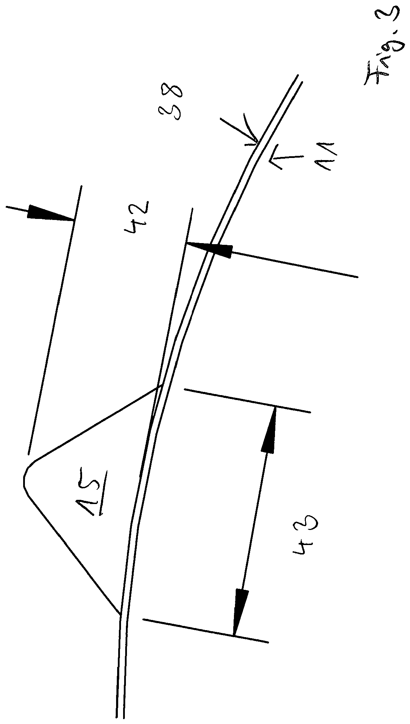

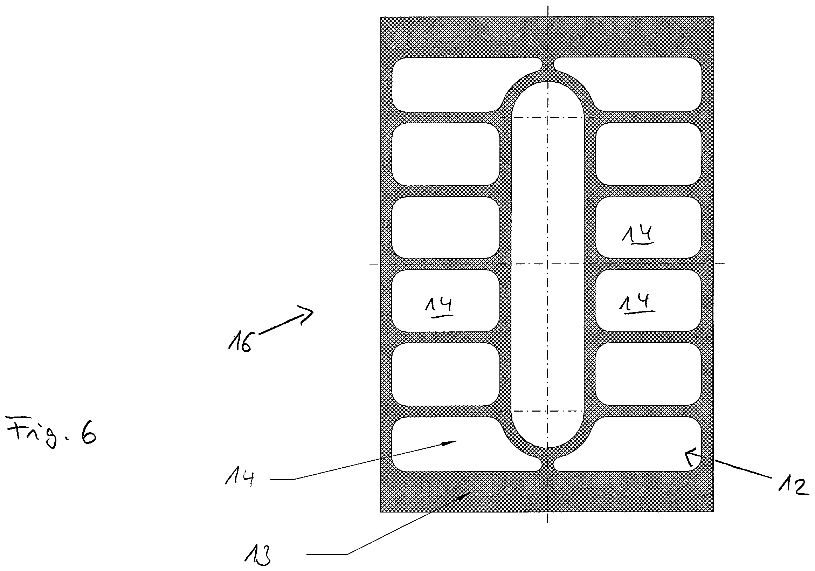

Fig. 1 zeigt eine erfindungsgemäße Vorrichtung 1 in perspektivischer Ansicht. Die Vorrichtung 1 dient zur Herstellung eines Komposits 16 umfassend Partikel und/oder Pulver 15, wie in Fig. 6 beispielhaft dargestellt mit Hilfe von Unterdruckbereichen 6, wie in Fig. 2 dargestellt. Die Partikel und/oder Pulver 15 umfassen insbesondere Absorberpartikel zur Aufnahme vorn Flüssigkeit, um ein Durchtropfen zu verhindern, z.B. als Fleischtuch, Hygieneeinlage und ähnliches. Das Komposit 16 kann beispielsweise auch als Pad zur Wirkstoffabgabe eingesetzt werden, wenn die eingeschlossenen Partikel und/oder Pulver Wirkstoff unter Flüssigkeitszugabe langsam abgeben können. Das erfindungsgemäße Komposit umfasst mindestens zwei Materiallagen 2, wobei das Material 4 beispielhaft Vlies sein kann, zwischen die in geschlossenen Kammern Partikel und/oder Pulver eingeschlossen ist, , wie in Fig. 6 dargestellt, wobei die Abgrenzung der Partikel und/oder Pulver so ausgebildet ist, dass an den Rändern der Kammern kein Partikel und/oder Pulver eingeschlossen wird, die dann zum unerwünschten Aufplatzen der Ränder beim Aufquellen führen können. Die Vorrichtung zur Herstellung des Komposits umfasst beispielhaft eine zumindest teilweise rotierbare Walze 5 mit Unterdruckbereichen 6, wie in Fig. 2 dargestellt. Die Walze 5 weist beispielhaft ein rotierendes Mantelmittel 3 mit einer Außenwand 11 auf und eine stationäre Welle 9 im Inneren. Durch eine beispielhafte Bohrung 40 kann ein Überdruck und/oder Unterdruck in die Welle 9 eingeleitet werden. Die Unterdruckbereiche 6 sind an der nicht drehbaren Welle 9 im Inneren der zumindest teilweise rotierenden Walze 5 angeordnet, wie beispielsweise in Fig. 2 dargestellt, und saugen somit an vorbeidrehenden Transportdüsen 10, die den Unterdruck vom Inneren an die Außenwand 11 befördern. Dort wird durch den Unterdruck die als Spuren 34 auf einem Lieferband 37 gelieferten Partikel und/oder Pulver 15 in Form von abgegrenzten Portionen mitgenommen, wie beispielhaft in Fig. 3 dargestellt. Die erste Materiallage 38 nimmt nur an der Stelle Partikel und/oder Pulver mit, an der das Vakuum anliegt.

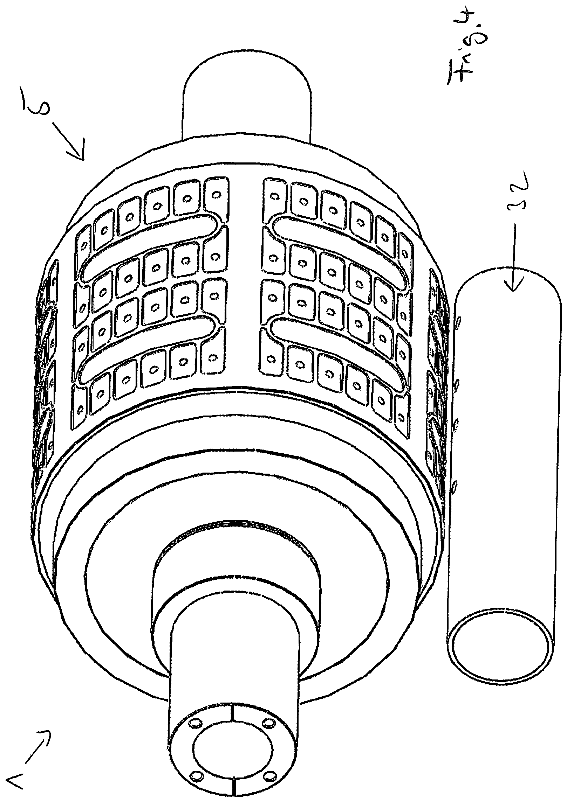

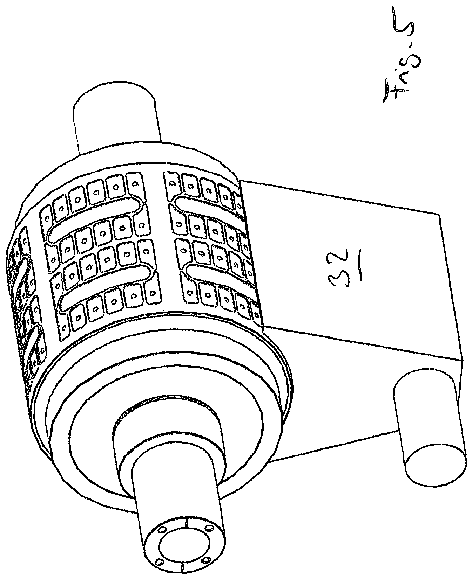

Die Partikel und/oder Pulver 15 kann der Walze 5 auf verschiedene Arten angeboten werden, wie in Fig. 1 beispielhaft als Lieferband 37 dargestellt mit einem Mittel zur Zuführung 32, das Partikel und/oder Pulver auf das Band aufbringt. Weitere Beispiele finden sich in Fig. 4 und Fig. 5.

Die Vorrichtung 1 umfasst weiterhin zur Belieferung von Material 4 in Form von Materiallagen 2 beispielhaft ein erstes 30 und ein zweites 31 Versorgungsmittel, wobei insbesondere die zweite Materiallage 39 durch eine Rolle 35 auf die Walze 5 geliefert werden. Eine erste Materiallage 38 wird in Rotationsrichtung 36 vor dem Mittel zur Zuführung 32 der Partikel und/oder Pulver angesetzt. Beispielhaft wird dazu von einer Rohmaterialrolle ein Vliesstoff abgewickelt und von unten um die Walze 5 geschlungen. Anschließend folgt auf das Mittel zur Zuführung 32 die zweite Materiallage 39 sowie ein Mittel zum Verbinden 33, welches beispielhaft ein Schweißverfahren, insbesondere eine Ultraschalleinheit, Temperaturschweißen oder ein Klebeverfahren, gemäß der Struktur der Walze 5 in Bereichen 13 versiegelt. Nach dem Versiegeln befindet sich Partikel und/oder Pulver in den verschlossenen Bereichen als Kammern 14 des Produktes. Das Komposit 16 als ein fertiges Produkt und/oder Zwischenprodukt kann dann beispielhaft mit Hilfe einer zweiten Rolle 35 von der Walze abtransportiert werden. Als folgender Schritt erfolgt das Aufwickeln der Komposit-Materialbahn. Hier kann auch direkt eine Vereinzelung erfolgen, oder die Produkte können anschließend durch z.B. einen Querschneider vereinzelt bzw. direkt weiter verarbeitet werden.

Die Partikel und/oder Pulver 15 werden somit durch die Vorrichtung 1 mittels des in einem vorbestimmten Anlagebereich zur Walze 5 angeordneten Mittels zur Zuführung 32 derart an vorbestimmten Bereichen 12 angeboten, dass durch den Unterdruck die vorbestimmten Bereiche 12 auf der Materiallage 38 mit Partikel und/oder Pulver 15 bedeckt und mittels eines zweiten, an der Walze 5 angeordneten Versorgungsmittels 31 eine zweite Materiallage 39 auf die erste Materiallage 38 mit Partikeln und/oder Pulver aufgedeckt werden, wobei diese in vorbestimmten Bereichen 13, die insbesondere zwischen den Unterdruckbereichen 6 entlanglaufen, mit der ersten Materiallage 38 durch das Einwirken des Mittels zum Verbinden 33 verbunden werden, so dass das Komposit 16 zumindest eine im Wesentlichen geschlossene Kammer 14 umfassend Partikel und/oder Pulver 15 aufweist.

Erfindungsgemäß wird somit die Partikel und /oder Pulver 15 der Walze 5 direkt angeboten und die Walze 5 führt verschiedene Aufgaben durch: Die erste Materiallage 38 zu transportieren, die Partikel und /oder Pulver 15 zu portionieren, die Kombination erste Materiallage 38 mit dem portioniertem und positioniertem Partikel und /oder Pulver 15 zu transportieren, auf dieser Kombination die zweite Materiallage 39 als Abdeckmaterial zu positionieren und zu transportieren, die erste Materiallage 38 mit dem Abdeckmaterial zu fixieren / zu verbinden, das Komposit 16 von der Vorrichtung 1 als Vakuumsystem zu lösen.

Fig. 2 zeigt eine Walze 5 einer Vorrichtung 1 in Explosionsdarstellung. Die Walze 5, an deren Außenwand 11 Strukturen mit Transportmitteln 7 und Transportdüsen 10 zum Aufbau der Kammern 14, beispielhaft in Fig. 6, des Komposits angeordnet sind, weist im Inneren ein Mittel zur Druck- und/oder Unterdruckerzeugung 23 auf, insbesondere eine Venturidüsenanordnung. Beispielhaft ist es im Inneren so aufgebaut, dass nur der Bereich mit Unterdruck/Vakuum beaufschlagt wird, der das Pulver halten soll. Im restlichen Bereich findet keine Vakuumbeaufschlagung statt, da dort entweder noch kein Partikel und/oder Pulver gehalten bzw. das Produkt fertig ist und von der Walze abgelöst werden soll. Die Walze 5 rotiert um die Welle 9, die fest steht. Die Walze 5 ist beispielhaft über ein Kugellager 8 gelagert und fest mit einem Antriebsmittel 41, insbesondere einem Zahnriemenrad, verbunden. Über das Zahnriemenrad erfolgt beispielhaft der Antrieb der Walze. Das Vakuum wird beispielhaft über eine externe Pumpe realisiert, die an einem Anschluss im Inneren angeschlossen ist und über eine innere Bohrung in der Welle 9 die Unterdruckbereiche 6 versorgt. Diese Unterdruckbereiche 6 wiederum beaufschlagen über die Transportdüsen 10 den entsprechenden Teil der Walze 5, wenn diese gerade an den Bereich vorbeirotiert. Die Versorgung mit Unterdruck kann beispielhaft auch über einen in der Walze eingebauten Vakuumerzeuger erfolgen beispielhaft Erzeugung des Vakuums mittels Druckluft nach dem Bernoulliprinzip in Form von Venturidüsen.

Fig. 3 zeigt eine Partikel- und/oder Pulveraufhäufung 15. Die Menge an Pulver, die über dieses Verfahren mitgenommen wird und als Pulverhäufchen 15 durch den Unterdruck der Walze 5 an der ersten Materiallage 38 anhaften, ist von verschiedenen Faktoren abhängig, insbesondere kann über den Bohrungsdurchmesser die Menge des Luftstroms und somit auch die Breite 43 der Pulverhäufchen beeinflusst werden. Weiterhin kann über den anliegende Unterdruck bzw. die Größe der angeschlossenen Vakuumerzeuger, die Höhe 42 der Pulverhäufchen beeinflusst werden. Je stärker der Luftstrom ist, desto höher werden die Pulverhäufchen. Je größer die Bohrungen in dem Mittel zur Zuführung 32 ausgeführt sind, desto mehr Pulver wird auf das Lieferband 37 abgelegt und desto breiter und höher werden die Pulverspuren. Da sich durch die heutige Servomotorentechnik die Geschwindigkeiten der Vakuumwalze im Verhältnis zum Transportband sehr gut regeln lassen, ist hier eine sehr genaue Dosierung pro Pulverhäufchen möglich.

Fig. 4 zeigt eine erfindungsgemäße Vorrichtung 1. Die Walze 5 ist beispielhaft mit einer Struktur für den Aufbau des Komposits 16 als Zwischen- und/oder Endprodukt versehen. Vakuum kann beispielhaft über Transportdüsen 10 als Vakuumbohrungen an den Stellen der Walze 5 gezogen werden, an der sich anschließend der Partikel und/oder Pulver 15, insbesondere Superabsorber, in den Kammern 14 des Produkts befinden soll. Die Partikel und/oder Pulver 15 wird hier beispielhaft in Form eines Mittels zur Zuführung 32 beispielhaft als Förderschnecke zugeführt. Die Förderschnecke weist an der Oberfläche Löcher zum Durchlass von Partikel und/oder Pulver im Bereich der Transportdüsen auf. Diese Löcher können auch als durchgehender Schlitz ausgeführt sein.

Fig. 5 zeigt eine erfindungsgemäße Vorrichtung 1 mit einem Mittel zur Zuführung 32 in Form eines Kastens mit Partikeln und/oder Pulver, der zur Walze hin offen ist.

Fig. 6 zeigt ein Komposit 16. In Bereichen 12 sind Partikel und/oder Pulver in Form von Kammern 14 eingeschlossen, in Bereichen 13 liegt die Befestigung. Die Vielfalt der möglichen Produkte, die mit diesem Verfahren herzustellen sind, reicht beispielhaft von Meatpads, wo beispielsweise Gewebeflüssigkeit in den Verkaufsschalen von Frischfleisch aufgefangen und gebunden wird, über den Hygienebereich, bei dem verschiedene Körperflüssigkeiten gebunden und gehalten werden bis in den Arzneimittelbereich, wobei beispielsweise Wirkstoff je nach Anwesenheit von Flüssigkeit abgeben wird, beispielsweise Gerinnungsmittel bei stark blutenden Wunden. Es kann beispielsweise auch andere rieselfähige Stoffe zugeführt werden, beispielsweise Tee, Kaffee, verschiedene Salze oder Medikamente in Pulverform sein.

Ebenso können verschiedene bahnförmige Materialien eingesetzt werden, die sich mittels Ultraschall oder Temperaturverfahren verschweißen lassen, beispielsweise Folien, Textilien, Vliesstoffe, wobei die zur Vakuumwalze gerichtete Seite vorteilhaft eine luftdurchlässige Materialbahn ist.

### BEZUGSZEICHENLISTE

- 1: Vorrichtung zur Herstellung eines Komposits
- 2: Materiallage
- 3: Mantelmittel
- 4: Material
- 5: Walze
- 6: Unterdruckbereich
- 7: Transportmittel
- 8: Kugellager
- 9: Welle
- 10: Transportdüsen
- 11: Außenwand
- 12: Bereich
- 13: Bereich
- 14: Kammer
- 15: Partikel und/oder Pulver
- 16: Komposit
- 23: Mittel zur Druck- und/oder Unterdruckerzeugung
- 30: erstes Versorgungsmittel
- 31: zweites Versorgungsmittel
- 32: Mittel zur Zuführung
- 33: Mittel zum Verbinden
- 34: Spur
- 35: Rolle
- 36: Rotationsrichtung
- 37: Lieferband
- 38: erste Materiallage
- 39: zweite Materiallage
- 40: Bohrung
- 41: Antriebsmittel
- 42: Höhe
- 43: Breite

## Patentansprüche

1. Vorrichtung (1) zur Herstellung eines Komposits (16) umfassend Partikel und/oder Pulver (15), insbesondere Absorberpartikel und/oder Partikel zur Wirkstoffabgabe , sowie mindestens zwei Materiallagen (2), umfassend eine zumindest teilweise rotierbare Walze (5) mit Unterdruckbereichen (6), ein erstes (30) und ein zweites (31) Versorgungsmittel, ein Mittel zur Zuführung (32), ein Mittel zum Verbinden (33), wobei auf der Walze (5) eine erste Materiallage (38) mittels des ersten Versorgungsmittels (30) aufzulegen ist und die Walze (5) an vorbestimmten Bereichen (12) mit Unterdruck beaufschlagt ist zum Ansaugen der Materiallagen (2) und/oder von Partikeln und/oder Pulver (15), die mittels des in einem vorbestimmten Anlagebereich zur Walze (5) angeordneten Mittels zur Zuführung (32) derart an den vorbestimmten Bereichen (12) anzubieten sind, dass durch den Unterdruck die vorbestimmten Bereiche (12) auf der Materiallage (38) mit Partikel und/oder Pulver (15) zumindest teilweise bedeckt sind und mittels eines zweiten, an der Walze (5) angeordneten Versorgungsmittels (31) eine zweite Materiallage (39) auf die erste Materiallage mit Partikeln und/oder Pulver (15) aufzudecken ist, wobei diese in vorbestimmten Bereichen (13), die insbesondere zwischen den Unterdruckbereichen (6) angeordnet sind, mit der ersten Materiallage insbesondere durch Einwirken des Mittels zum Verbinden (33), das an der Walze (5) angeordnet ist, verbunden ist, so dass das Komposit (16) zumindest eine im Wesentlichen geschlossene Kammer (14) umfassend Partikel und/oder Pulver (15) aufweist,
wobei die zumindest teilweise rotierende Walze (5) eine insbesondere feststehende Welle (9) aufweist, wobei die Partikel und/oder Pulver (15) und/oder die Materiallage (2), insbesondere abgetrennt von und/oder ausgestanzt aus flächigem, insbesondere bahnförmigem Material (4), mittels eines der Welle (9) zugeordneten Mittels (23) zur Druck- und/oder Unterdruckerzeugung beaufschlagt ist, nämlich einem Mittel unter Ausnutzung des Bernoullieffekts, nämlich einer oder mehrerer Venturidüsen, direkt oder indirekt durch Transportmittel (7), insbesondere Transportdüsen (10), zur Druckweiterleitung aus dem Bereich der Welle (9), insbesondere an dem Unterdruckbereich (6), durch ein rotierendes Mantelmittel (3) hindurch manipuliert sind, so dass insbesondere in einem vorbestimmten lokalen Bereich (12) des Mantelmittels (3) die Materiallage (2) und/- oder Partikel und/oder Pulver (15) mittels Unterdruck festgehalten und/oder mittels Überdruck abgestoßen werden.

2. Vorrichtung nach Anspruch 1, **dadurch gekennzeichnet, dass** die Walze (5) am Mantelmittel (3) mit einer Struktur versehen ist, die die Begrenzungen der Kammern mit den Partikeln und/der Pulver (15) ausformen.

3. Vorrichtung nach einem der Ansprüche 1 bis 2, **dadurch gekennzeichnet, dass** das erste (30) und/oder zweite (31) Versorgungsmittel Rollen (35) zum Abrollen der Materiallagen (2) umfassen.

4. Vorrichtung nach einem der Ansprüche 1 bis 3, **dadurch gekennzeichnet, dass** das Mittel zur Zuführung (32) für die Partikel und/oder das Pulver (15) hinsichtlich der Rotationsrichtung (36) nach dem Auflegen der ersten Materiallage und vor dem Auflegen der zweiten Materiallage angeordnet ist und/oder das Mittel zum Verbinden (33) hinsichtlich der Rotationsrichtung (36) der Walze (5) nach dem Aufsetzpunkt der zweiten Materiallage angeordnet ist.

5. Vorrichtung nach einem der Ansprüche 1 bis 4, **dadurch gekennzeichnet, dass** das Mittel zur Zuführung (32) ein Lieferband (37) umfasst, das die Partikel und/oder das Pulver (15) in einer Spur (34) zu der Walze (5) transportiert, wobei durch eine Mittel zur Zuführung (32) insbesondere eine vorbestimmte bahnartige Besetzung des Lieferbandes (37) vorzunehmen ist.

6. Vorrichtung nach einem der Ansprüche 1 bis 5, **dadurch gekennzeichnet, dass** das Mittel zur Zuführung (32) eine insbesondere kastenartige Befülleinrichtung umfasst, die unterhalb der Walze (5) angerordnet ist, wobei der Unterdruck Partikel und/oder Pulver (15) aus der Befülleinrichtung herausheben kann und/oder das Mittel zur Zuführung (32) eine Förderschnecke umfasst, die insbesondere an einer Oberfläche Löcher im Bereich von Transportdüsen aufweist, wobei diese Löcher auch als durchgehender Schlitz ausgeführt sein können.

7. Vorrichtung nach einem der Ansprüche 1 bis 6, **dadurch gekennzeichnet, dass** das Mittel zum Verbinden (33) eine Klebeeinrichtung und/oder eine Einrichtung zum Ultraschallschweißen und/oder eine Temperaturschweißvorrichtung umfasst.

8. Vorrichtung nach einem der Ansprüche 1 bis 7, **dadurch gekennzeichnet, dass** die Materiallage (2) umfasst Polymer und/oder Vlies und/oder Folie und/oder Gewebe und/oder Gewirke und/oder ähnliche Flächengebilde, die insbesondere zur Weiterleitung von Druckänderungen geeignet sind und/oder flüssigkeitsdurchlässig sind.

9. Verfahren zur Herstellung eines Komposits (16) umfassend Partikel und/oder Pulver (15), insbesondere Absorberpartikel, insbesondere nach einem der Ansprüche 1 bis 8, sowie mindestens zwei Materiallagen (2), wobei eine Materiallage auf einer zumindest teilweise rotierenden Walze (5) mit vorbestimmten Unterdruckbereichen (6) bereitgestellt wird und diesen Unterdruckbereichen (6) Partikel und/oder Pulver (15) zum Ansaugen durch den Unterdruck mittels eines Mittel zur Zuführung (32) angeboten werden, so dass durch den Unterdruck die vorbestimmten Bereiche auf der Materiallage (2) mit Partikeln und/oder Pulver (15) im Wesentlichen bedeckt werden und anschließend mittels eines zweiten Versorgungsmittels eine zweite Materiallage auf die erste Materiallage mit den Partikeln und/oder Pulver (15) aufgelegt wird und in vorbestimmten Bereichen (12), die insbesondere zwischen den Unterdruckbereichen (6) angeordnet sind, mit der ersten Materiallage insbesondere durch Einwirken eines Mittels zum Verbinden (33), das an der Walze (5) angeordnet ist, verbunden werden, so dass zumindest eine oder mehrere im Wesentlichen geschlossene Kammer (14) umfassend Partikel und/oder Pulver (15) hergestellt wird,
wobei die zumindest teilweise rotierende Walze (5) eine insbesondere feststehende Welle (9) aufweist, wobei die Partikel und/oder Pulver (15) und/oder die Materiallage (2), insbesondere abgetrennt von und/oder ausgestanzt aus flächigem, insbesondere bahnförmigem Material (4), mittels eines der Welle (9) zugeordneten Mittels (23) zur Druck- und/oder Unterdruckerzeugung beaufschlagt ist, nämlich einem Mittel unter Ausnutzung des Bernoullieffekts, nämlich einer oder mehrerer Venturidüsen, direkt oder indirekt durch Transportmittel (7), insbesondere Transportdüsen (10), zur Druckweiterleitung aus dem Bereich der Welle (9), insbesondere an dem Unterdruckbereich (6), durch ein rotierendes Mantelmittel (3) hindurch manipuliert sind, so dass insbesondere in einem vorbestimmten lokalen Bereich (12) des Mantelmittels (3) die Materiallage (2) und/- oder Partikel und/oder Pulver (15) mittels Unterdruck festgehalten und/oder mittels Überdruck abgestoßen werden.

## Claims

1. Device (1) for making a composite (16) comprising particles and/or powder (15), in particular absorber particles and/or active substance release particles, and at least two layers of material (2), comprising at least a partially rotating roller (5) with negative pressure ranges (6), a first (30) and a second (31) supply, a means for feeding (32), a means for joining (33), with on the roller (5) a the first layer of material (38) must be applied by means of the first supply (30) and the roller (5) must be ressurized at predetermined areas (12) for suction of the layers of material (2) and/or particles and/or powder (15), the supply medium (32) located in a pre-defined area of the roller (5) is to be provided to the pre-defined areas (12) in such a way that the pre-defined areas (12) on the material layer (38) are at least partly covered by particles and/or powder (15) by the negative pressure and by a second supply placed on the roller (5) by means of (31) a second layer of material (39) on the first layer of material with particles and/or powder (15), where these are located in pre-defined areas (13) arranged in particular between the vacuum areas (6) with the first layer of material, in particular by the action of the bonding agent (33) arranged on the roller (5), so that the composite (16) contains at least one substantially closed chamber (14) comprising particles and/or powder (15) at least partly rotary roller (5) has a particularly fixed shaft (9), where the particles and/or powder (15) and/or the material layer (2), in particular separated from and/or stamped from flat material, in particular web-shaped material (4), are charged by means of a means (23) assigned to shaft (9) to produce pressure and/or vacuum, i. e. a means exploiting the bernoullie effect, i. e. one or more Venturi nozzles, directly or indirectly by means of transport (7), in particular transport nozzles (10), for the transmission of pressure from the area of the shaft (9) are manipulated in particular at the vacuum area {6}, through a rotating coating agent (3) so that, in particular in a predetermined local area {12) of the coating agent (3), the material position (2) and/or particles and/or powder (15) are held by vacuum and/or repelled by overpressure.

2. Device according to Claim 1, **characterised by** the fact that the roller (5) on the coating medium (3) is provided with a structure forming the boundaries of the chambers containing particles and/or powder (15).

3. Device according to one of Claims 1 to 2, **characterised in that** the first (30) and/or second (31) supply includes rolls (35) for rolling the layers of material (2).

4. Device according to one of Claims 1 to 3, **characterised by** the arrangement of the feeder (32) for the particles and/or powder (15) with respect to the direction of rotation (36) after the laying of the first layer of material and before the laying of the second layer of material and/or the connection (33) with respect to the direction of rotation (36) of the roller (5) with respect to the point of attachment of the second layer of material is arranged.

5. Device according to one of Claims 1 to 4, **characterised by** the fact that the means of feeding (32) comprises a delivery belt (37) which transports the particles and/or powder (15) in a trace (34) to the roller (5), whereby a means of feeding (32) in particular a predetermined rail-like filling of the delivery belt (37) is to be carried out by means of a means of feeding (32).

6. Device according to one of Claims 1 to 5, **characterised by** the fact that the feeding device (32) comprises a particularly container-like filling device located below the roller (5), the vacuum being capable of lifting particles and/or powder (15) out of the feeding device and/or the feeding device (32) comprises a screw conveyor, in particular has holes on a surface in the area of transport nozzles, which may also be made as a continuous slot.

7. Device according to one of Claims 1 to 6, **characterised by** the fact that the connecting medium (33) includes an adhesive device and/or an ultrasonic welding device and/or a temperature welding device.

8. Device according to one of Claims 1 to 7 **characterised by** the fact that the material layer (2) comprises polymer and/or non-woven and/or foil and/or fabrics and/or knitted fabrics and/or similar surfaces which are particularly suitable for transmitting changes in pressure and/or are permeable to liquids.

9. Method for producing a composite (16) comprising particles and/or powder (15), in particular absorber particles, in particular according to one of Claims 1 to 8, and at least two layers of material (2), providing a layer of material on at least a partially rotating roller (5) with predetermined vacuum zones (6) and to these vacuum zones (6) particles and/or powder (15) for suction by the vacuum a means of feeding (32) is offered so that the vacuum essentially covers the predetermined areas of the material layer (2) with particles and/or powder (15) and then, by means of a second supply, a second layer of material is applied to the first layer of material containing particles and/or powder (15) and in predetermined areas (12), which are located, in particular, between the vacuum areas (6), are connected to the first layer of material, in particular by the action of a bonding agent (33) located on the roller (5) to produce at least one or more substantially closed chamber (14) containing particles and/or powder (15), where the roller (5), which is at least partly rotating, has a particularly fixed shaft (9), whereby the particles and/or powder (15) and/or the material layer (2), in particular separated from and/or punched from flat material, in particular web-shaped material (4), are applied by means of a means assigned to the shaft (9), to generate pressure and/or vacuum is a means using the bernoullie effect, namely one or more venturi nozzles, manipulated directly or indirectly by means of transport, in particular transport nozzles (10), for the transmission of pressure from the area of the shaft (9), in particular from the area of the vacuum (6), through a rotating sheathing medium (3), so that, in particular, in a predetermined local area (12) of the sheath by means of (3) the material (2) and/or particles and/or powder (15) are held by vacuum and/or repelled by overpressure.

## Revendications

1. Dispositif (1) pour la fabrication d'un composite (16) comprenant des particules et/ou des poudres (15), en particulier des particules absorbantes et/ou des particules destinées au transfert de la substance active, et au moins deux matériaux (2), comprenant un rouleau au moins partiellement rotatif (5) avec des zones de dépression (6), un premier (30) et un deuxième (31) aliment, un aliment d'alimentation (32), un agent de liaison (33), et sur la baleine (5) une première épaisseur de matériau (38) est mise en place au moyen du premier aliment (30) et le rouleau (5) est soumis à une dépression dans des zones prédéterminées (12) afin d'aspirer les matériaux (2) et/ou les particules et/ou les poudres (15), les moyens d'alimentation (32) disposés dans une zone prédéterminée d'installation sur le rouleau (5) doivent être proposés dans les zones prédéterminées (12) de telle sorte que la dépression recouvre au moins partiellement les zones prédéterminées (12) sur la couche de matériau (38) de particules et/ou de poudre (15) et par un second versant placé sur le rouleau (5). dans l'aliment (31), une seconde couche (39) est décelée à la première couche de particules et/ou de poudres (15), dans des zones prédéterminées (13), disposées notamment entre les zones de dépression (6), elles sont reliées à la première couche de matériau, notamment par l'action de l'agent de liaison (33), placé sur le rouleau (5), de sorte que le composite (16) comporte au moins une chambre essentiellement fermée (14) contenant des particules et/ou des poudres (15), la un rouleau tournant au moins partiellement (5) comporte un arbre particulièrement fixe (9), où les particules et/ou les poudres (15) et/ou l'état du matériau (2) sont, en particulier, isolés et/ou découpés de matériaux superficiels, notamment en forme de rails (4), soumis à un traitement de pression et/ou de dépression par un agent (23) associé à l'arbre (9), à savoir un agent utilisant l'effet de Bernoullie, à savoir un ou plusieurs buses de Venturidis, directement ou indirectement par des moyens de transport (7), notamment des buses de transport (10), pour la transmission de la pression à partir de la zone de l'arbre (9). sont manipulés, notamment dans la zone de dépression (6), par un agent d'enveloppe rotatif (3), de telle sorte que, notamment dans une zone locale prédéterminée (12) du produit d'enveloppe (3), la position du matériau (2) et/ou les particules et/ou les poudres (15) soient retenues par dépression et/ou rejetées par surpression.

2. Dispositif selon la revendication 1, **caractérisé par le fait que** le rouleau (5) sur le revêtement (3) est pourvu d'une structure qui forme les limites des chambres contenant les particules et/ou les poudres (15).

3. Dispositif selon l'une des revendications 1 à 2, **caractérisé par le fait que** le premier (30) et/ou le second (31) aliment comporte des rouleaux (35) pour dérouler les matériaux (2).

4. Dispositif selon l'une des revendications 1 à 3, **caractérisé par le fait que** le moyen d'alimentation (32) des particules et/ou de la poudre (15) est disposé par rapport au sens de rotation (36) après l'application de la première couture et avant l'application de la seconde couture et/ou le moyen de liaison (33) par rapport au sens de rotation (36) du rouleau (5) par rapport au point de pose de la seconde couture L'état des matériaux est disposé.

5. Dispositif selon l'une des revendications 1 à 4, **caractérisé par le fait que** le moyen d'alimentation (32) comporte une bande d'alimentation (37) qui transporte les particules et/ou la poudre (15) sur une voie (34) jusqu'au laminoir (5), avec un moyen d'alimentation (32), notamment un remplissage ferroviaire prédéterminé de la bande d'alimentation (37).

6. Dispositif selon l'une des revendications 1 à 5, **caractérisé par le fait que** le moyen d'alimentation (32) comprend un dispositif de remplissage particulier de type caisson, placé sous le rouleau (5), dans lequel la dépression peut faire sortir des particules et/ou de la poudre (15) du dispositif de remplissage et/ou le moyen d'alimentation (32) est muni d'une conduite La surface comporte notamment des trous dans la zone des buses de transport, ces trous pouvant également être réalisés en fente continue.

7. Dispositif selon l'une des revendications 1 à 6, **caractérisé par le fait que** le moyen de liaison (33) comprend un dispositif de collage et/ou un dispositif de soudage par ultrasons et/ou un dispositif de soudage thermique.

8. Dispositif selon l'une des revendications 1 à 7, **caractérisé par le fait que** le matériau (2) comprend des polymères et/ou des toiles et/ou des feuilles et/ou des tissus et/ou des tricots et/ou des surfaces similaires, qui sont particulièrement aptes à transmettre les changements de pression et/ou qui sont perméables aux fluides.

9. Procédé de fabrication d'un composite (16) comprenant des particules et/ou de la poudre (15), en particulier des particules absorbantes, en particulier selon l'une des revendications 1 à 8, et au moins deux matériaux (2), en prévoyant une couche de matériau sur un rouleau rotatif au moins partiellement (5) avec des plages de dépression prédéterminées (6) et en utilisant ces plages de pression (6) des particules et/ou de la poudre (15) pour l'aspiration par le La sous-pression recouvre essentiellement les zones prédéterminées de la couche de matériau (2) de particules et/ou de poudre (15), puis, au moyen d'un second aliment, une seconde couche de matériau est appliquée à la première couche de particules et/ou de poudre (15) et dans des conditions prédéterminées. domaines (12), disposés notamment entre les zones de dépression (6) et reliés à la première position des matériaux, notamment par l'action d'un agent de liaison (33) placé sur le rouleau (5), de manière à produire au moins une ou plusieurs chambres essentiellement fermées (14) contenant des particules et/ou des poudres (15) le rouleau tournant au moins en partie (5) comporte un arbre particulièrement fixe (9), dans lequel les particules et/ou la poudre (15) et/ou l'état du matériau (2), en particulier séparés et/ou découpés de matériaux superficiels, en particulier en forme de rails (4), au moyen d'un agent de pression et/ou de dépression associé à l'arbre (9) (23) la production (23) est réglementée, c'est-à-dire un agent utilisant l'effet de Bernoullie, à savoir une ou plusieurs buses de Venturidis, manipulées directement ou indirectement par des moyens de transport, notamment des buses de transport (10), pour la conduction de la pression à partir de la zone de l'arbre (9), notamment dans la zone de dépression (6), par un agent d'enveloppe rotative (3), de telle sorte que, notamment dans une zone locale prédéterminée (12) du En ce qui concerne le produit d'enveloppe (3), la position du matériau (2) et/ou les particules et/ou les poudres (15) sont maintenues par dépression et/ou rejetées par surpression.
